(19) Europäisches Patentamt
European Patent Office
Office européen des brevets

(11) Veröffentlichungsnummer: **0 172 446**
**A2**

(12) # EUROPÄISCHE PATENTANMELDUNG

(21) Anmeldenummer: 85109433.4

(22) Anmeldetag: 26.07.85

(51) Int. Cl.⁴: A 61 K 31/415
A 61 K 31/135, A 61 K 31/5-
A 61 K 31/165

(30) Priorität: 02.08.84 DE 3428525

(43) Veröffentlichungstag der Anmeldung:
26.02.86 Patentblatt 86/9

(84) Benannte Vertragsstaaten:
AT BE CH DE FR GB IT LI LU NL SE

(71) Anmelder: Boehringer Mannheim GmbH
Sandhofer Strasse 116
D-6800 Mannheim 31(DE)

(72) Erfinder: Böhm, Erwin, Dr. med.
In der Schanz 37
D-6905 Schriesheim(DE)

(72) Erfinder: Hölck, Jens-Peter, Dr. rer. nat.
Ph. Brunnemer Weg 33
D-6800 Mannheim 31(DE)

(72) Erfinder: Kampe, Wolfgang, Dr. rer. nat.
Zedernstrasse 49
D-6805 Heddesheim(DE)

(72) Erfinder: Leinert, Herbert, Dr.
Essigkamm 11
D-6148 Heppenheim(DE)

(72) Erfinder: Müller-Beckmann, Bernd, Dr.
Hochgewanne 46
D-6718 Grünstadt(DE)

(72) Erfinder: Strein, Klaus, Prof.
Eichenstrasse 45
D-6944 Hemsbach(DE)

(54) **Verwendung von Alkylendiamin-Derivaten zur Behandlung von Durchblutungsstörungen sowie Arzneimittel, die diese Verbindungen enthalten.**

(57) Verwendung von Verbindungen der Formel I

$$Ⓐ-O-CH_2-\underset{}{\overset{OH}{\overset{|}{C}H}}-CH_2-\underset{\underset{R_8}{|}}{N}-X-\underset{\underset{R_9}{|}}{N}-Y-Ⓑ \qquad (I)$$

in der

A einen gegebenenfalls substituierten Phenyl- oder heteroaromatischen Rest,
$R_6$, $R_7$ Wasserstoff oder Niederalkyl,
X eine Alkylenkette mit bis zu 6 Kohlenstoffatomen,
Y einen Valenzstrich oder die >C=O-Gruppe und B einen gegebenenfalls substituierten Phenyl- oder heteroaromatischen Rest

bedeuten, sowie deren pharmakologisch verträglichen Salze zur Behandlung von Durchblutungsstörungen sowie Arzneimittel, die diese Verbindungen enthalten.

BOEHRINGER MANNHEIM GMBH 2684

# Verwendung von Alkylendiamin-Derivaten zur Behandlung von Durchblutungsstörungen sowie Arzneimittel, die diese Verbindungen enthalten

In den deutschen Offenlegungsschriften 28 19 629, 28 22 473, 28 44 497, 30 23 369 und 31 31 146 sind Alkylendiamine beschrieben, die an einem Stickstoffatom eine Aryloxypropanol-(2)-Gruppe und an dem anderen Stickstoffatom eine Aryl- oder Carbamoyl-Gruppe tragen, mit einer mehr oder weniger ausgeprägten cardiotonen Wirkung.

So ist z.B. das 1-Phenoxy-3-/2-(1,3,5-trimethyl-pyrazol-4-yl-amino)ethylamino7-propan-2-ol aus der DE-OS 30 23 369 eine Verbindung, die auf ß-Rezeptoren wirkt und das Herzzeitvolumen erhöht.

Periphere bzw. cerebrale Durchblutungsstörungen können über diese bewiesenen Effekte - Steigerung des Herzminutenvolumens, Stimulation oder Blockade der ß-Rezeptoren - allein nicht erfolgreich behandelt werden.

Anerkanntes Therapieprinzip für Durchblutungsstörungen ist jedoch die Verbesserung der Fließeigenschaften des Blutes, insbesondere die Verformbarkeit der Erythrozyten.

Es liegt diesem Sachverhalt die Tatsache zugrunde, daß die Erythrozyten bei ihrer Passage durch die Kapillaren sich stark verformen müssen, da der Erythrozyten-Durchmesser ca 7 µm beträgt, die Kapillar-Durchmesser aber bis herunter auf 3 µm gehen.

Patienten mit Durchblutungsstörungen, peripher, cerebral und auch coronar, haben in der Regel schlechter verformbare Erythrozyten. Hinzu kommt, daß die Erythrozyten bei der Passage durch das mangeldurchblutete Gebiet mit Stoffwechselprodukten wie etwa Milchsäure in Kontakt kommen, von denen bekannt ist, daß sie die Verformbarkeit der Erythrozyten weiter herabsetzen. Dies führt letztlich dazu, daß Erythrozyten regelrecht in den Kapillaren steckenbleiben können und damit die Endstrombahn für eine weitere Durchblutung verlegen.

Es wurde nun überraschenderweise gefunden, daß Alkylendiamine aus den eingangs genannten Druckschriften eine Erhöhung der Verformbarkeit der Erythrozyten bewirken und daher gezielt in der Therapie cerebraler, coronarer und peripherer Durchblutungsstörungen eingesetzt werden können.

Gegenstand der Erfindung ist die Verwendung von Verbindungen der allgemeinen Formel I

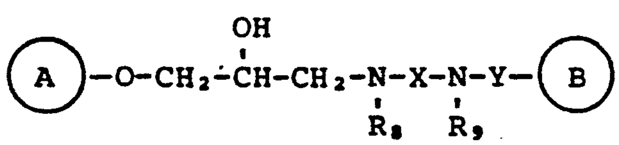

(I),

in der

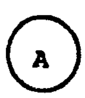 entweder

1. einen Phenylrest der allgemeinen Formel II

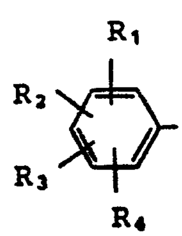

(II),

in welcher $R_1$, $R_2$, $R_3$ und $R_4$ gleich oder verschieden sind und Wasserstoff, Niederalkyl, Cyan, Carboxamido, Halogen, Hydroxy, Niederacyloxy, Niederalkoxy, Methoxyniederalkoxy, Niederalkenyloxy, Phenylniederalkoxy, Amino oder Niederacylamino bedeuten

oder

2. einen bi- oder tricyclischen, gegebenenfalls teilweise hydrierten heteroaromatischen Rest der allgemeinen Formel III

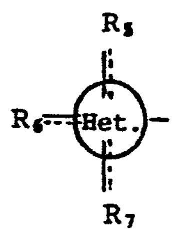

(III),

in welcher $R_5$, $R_6$, $R_7$ gleich oder verschieden sind und Wasserstoff, Niederalkyl, Benzyl, Niederalkanoyl, Cyan, Hydroxymethyl, Niederalkoxycarbonyl, Carbamoyl und die zweibindigen Reste Sauerstoff oder Schwefel bedeuten,

$R_8$, $R_9$ gleich oder verschieden sind und Wasserstoff oder Niederalkyl bedeuten,

X eine geradkettige oder verzweigte Alkylenkette mit bis zu 6 Kohlenstoffatomen ist,

Y ein Valenzstrich oder die >C=O-Gruppe ist und

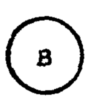 entweder

1. einen Phenylrest der allgemeinen Formel IV

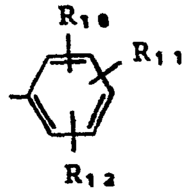

(IV),

in welcher $R_{10}$, $R_{11}$ und $R_{12}$ gleich oder verschieden sind und Wasserstoff, Halogen, Niederalkyl, Niederalkoxy, Niederalkylmercapto, Nitro, Amino, Niederacylamino oder $R_{10}$ und $R_{11}$ gemeinsam eine gegebenenfalls ungesättigte Trimethylenkette oder eine Alkylendioxy-Gruppe bedeuten

oder

2. einen mono-, bi- oder tricyclischen heteroaromatischen oder hydroheteroaromatischen Rest der allgemeinen Formel V

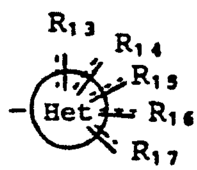

(V),

in welcher $R_{13}$, $R_{14}$, $R_{15}$, $R_{16}$, $R_{17}$ gleich oder verschieden sind und ein- oder zweibindige Substituenten bedeuten, und zwar Wasserstoff, Halogen, Niederalkyl, Allyl, Niederalkoxy, Allyloxy, Nitro, Amino, Niederacylamino, Cyano, Phenylniederalkyl, gegebenenfalls substituiertes Phenyl, Sauerstoff oder Schwefel,

oder

3. einen Aminrest der allgemeinen Formel VI

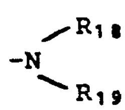

(VI),

in welcher $R_{18}$ und $R_{19}$ gleich oder verschieden sind und Wasserstoff, Niederalkyl, Hydroxyniederalkyl, Alkoxyniederalkyl, Phenyl oder gemeinsam mit dem Stickstoffatom einen gegebenenfalls durch Heteroatome wie Sauerstoff, Schwefel oder die Gruppe $>N-R_{20}$ unterbrochenen Ring,

$R_{20}$ Niederalkyl, gegebenenfalls substituiertes Phenyl, Niederalkanoyl oder gegebenenfalls substituiertes Benzoyl

darstellen

und ihre pharmakologisch verträglichen Salze zur Behandlung von Durchblutungsstörungen sowie Arzneimittel, die diese Verbindungen enthalten.

Niederalkyl bedeutet in allen Fällen einen Rest mit 1 - 6, vorzugsweise 1 - 4 C-Atomen, insbesondere Methyl und Ethyl.

Niederalkoxy bedeutet in allen Fällen, auch bei zusammengesetzten Resten, eine Gruppe mit 1 - 6, vorzugsweise 1 - 4 C-Atomen, insbesondere die Methoxy- und Ethoxy-Gruppe.

Niederalkylmercapto bedeutet $C_1$-$C_6$-Alkylmercapto, insbesondere Methylmercapto.

Niederacyl, allein oder bei zusammengesetzten Resten, bedeutet $C_1$-$C_6$-Acyl, wie Formyl, Acetyl, Propionyl und Butyryl.

Niederalkenyloxy bedeutet vorzugsweise Allyloxy.

Eine Alkylendioxy-Gruppe, die $R_{10}$ und $R_{11}$ zusammen bilden können, ist vorzugsweise die $-O-CH_2-O-$Gruppe.

Aryl bzw. Aroyl können übliche Substituenten wie Halogen,

Hydroxy, Niederalkyl, Niederalkoxy, Nitro oder Amino tragen.

Cyclische Gruppen, die gemäß Formel VI durch den Stickstoff und den Resten $R_{18}$ und $R_{19}$ gebildet werden können, sind bevorzugt Piperidin, gegebenenfalls substituiertes Piperazin und Morpholin.

Halogen bedeutet Fluor, Chlor, Brom und Jod, insbesondere Fluor und Chlor.

Bi- oder tricyclische heteroaromatische Reste A sind beispielsweise Indol, Indolizin, Isoindol, Benztriazol, Indazol, Purin, Chinolizin, Isochinolin, Chinolin, Chinoxalin, Chinazolin, Cinnolin, Carbolin, Carbazol, Acridin, Benzthiadiazol, Phenazin, Benzimidazol, vorzugsweise Indol, Benzimidazol, Indazol, Benztriazol, Carbazol, als teilweise hydrierter heteroaromatischer Rest beispielsweise Indolin, Isoindolin, Pyrrolin, Imidazolin, vorzugsweise Indolin.

Mono-, bi- oder tricyclische heteroaromatische Reste B sind beispielsweise die folgenden heteroaromatischen Reste: Pyrrol, Thiophen, Oxazol, Isoxazol, Thiazol, Pyrazol, Imidazol, 1.2.4-Triazol, Pyridin, Pyrimidin, Pyridazin, Pyrazin, 1.2.4-Triazin, 1.3.5-Triazin, Thionaphthen, Indol, Isoindol, Benzoxazol, Benzthiazol, 1.2-Benzisothiazol, Benzimidazol, Indazol, Benztriazol, Chinolin, Isochinolin, Chinazolin, Cinnolin, Chinoxalin, Phthalazin, Carbazol, ß-Carbolin, Pyrazolo[3.4-b]pyridin, Pyrazolo[3.4-d]pyrimidin und Purin, sowie entsprechende daraus abgeleitete hydroheteroaromatische Reste, wobei die Verknüpfung mit der

-Y-N-Kette
   |
   $R_9$

an einem Ring-Kohlenstoffatom oder auch an einem Ringstickstoffatom der betreffenden Heterocyclen erfolgen kann.

Hydro-heteroaromatische Reste A im Sinne der Erfindung sind teilweise hydrierte Derivate von bi- und tricyclischen Heterocyclen, beispielsweise Indolin, 1.2.3.4-Tetrahydrocarbazol, nicht aber durchhydrierte monocyclische Heterocyclen wie Pyrrolidin, Piperidin.

Die Kette X ist bevorzugt eine Ethylen- oder Propylen-Kette.

Bevorzugte erfindungsgemäße Verbindungen der Formel I sind Verbindungen, bei denen

A einen Phenyl-Rest, der gegebenenfalls durch Hydroxy substituiert ist oder einen Indazol-, Indol-, Benzimidazol- oder Benztriazol-Rest,

$R_8$, $R_9$ Wasserstoff oder Methyl,

X Ethylen,

Y Valenz oder eine >CO-Gruppe und

B einen Phenyl-Rest, der gegebenenfalls durch Methylgruppen substituiert ist, einen Pyrazol-Rest, der ein- bis dreifach durch Methyl, Propyl oder Allyl substituiert sein kann, oder
einen Pyrimidin-2.4-dion-Rest, der ein- bis dreifach durch Methyl substituiert sein kann,

bedeuten.

Insbesondere sind die folgenden Verbindungen bevorzugt:

a) 1-Phenoxy-3-[2-(1.3.5-trimethyl-pyrazol-4-yl-amino)-ethylamino]-propan-2-ol

b) 1-(4-Hydroxyphenoxy)-3-[2-(morpholincarbonamido)-ethylamino]-propan-2-ol

c) 1-Phenoxy-3-[2-(1.3.5-trimethyl-pyrimidin-2.4-dion-6-yl-N-methylamino)ethylamino]-propan-2-ol

d) 1-Phenoxy-3-[2-(1-allyl-3.5-dimethyl-pyrazol-4-yl-amino)-ethylamino]-propan-2-ol

e) 1-Phenoxy-3-[2-(1-n-propyl-3.5-dimethyl-pyrazol-4-yl-amino)-ethylamino]-propan-2-ol

f) 1-(4-Hydroxyphenoxy)-3-[2-(1.3.5-trimethyl-pyrazol-4-yl-amino)-ethylamino]-propan-2-ol

g) 1-Phenoxy-3-[2-(1.4-dimethyl-pyrazol-5-yl-amino)-ethylamino]-propan-2-ol

h) 1-(Indazol-4-yloxy)-3-[2-(2.6-dimethylphenylamino)-ethylamino]-propan-2-ol

Einige der beanspruchten Verbindungen sind neu. Sie können nach den in der eingangs genannten Druckschrift beschriebenen Verfahren hergestellt werden.

Zur Herstellung von Arzneimitteln werden die Substanzen der Forme I in an sich bekannter Weise mit geeigneten pharmazeutischen Traegersubstanzen, Aroma-, Geschmacks- und

Farbstoffen gemischt und beispielsweise als Tabletten oder Dragees ausgeformt oder unter Zugabe entsprechender Hilfsstoffe in Wasser oder Oel, wie z.B. Olivenoel, suspendiert oder geloest.

Die erfindungsgemaessen Substanzen der Formel I und ihre Salze koennen in fluessiger oder fester Form enteral oder parenteral appliziert werden. Als Injektionsmedium kommt vorzugsweise Wasser zur Anwendung, welches die bei Injektionsloesungen ueblichen Zusaetze wie Stabilisierungsmittel, Loesungsvermittler oder Puffer enthaelt. Derartige Zusaetze sind z.B. Tartrat- und Citratpuffer, Ethanol,Komplexbildner (wie Ethylendiamintetraessigsaeure und deren nichttoxische Salze), hochmolekulare Polymere (wie fluessiges Polyethylenoxyd) zur Viskositaetsregulierung. Feste Traegerstoffe sind z.B. Staerke, Lactose, Mannit, Methylcellulose, Talkum, hochdisperse Kieselsaeuren, hochmolekulare Fettsaeuren (wie Stearinsaeure), Gelatine, Agar-Agar, Calciumphosphat, Magnesiumstearat, tierische und pflanzliche Fette und feste hochmolekulare Polymere (wie Polyethylenglykole). Fuer orale Applikation geeignete Zubereitungen koennen gewuenschtenfalls Geschmacks- und Sueßstoffe enthalten.

Die beim Menschen anzuwendende Dosis hängt ab von Alter, Gewicht und Allgemeinbefinden des Patienten, der Schwere der Erkrankung, der Natur gleichzeitiger sonstiger Behandlungen, der Einnahme-Häufigkeit und der Natur der beabsichtigten Wirkung. Gewöhnlich beträgt die Tagesdosis der aktiven Substanz o.1 bis 5o mg pro /kg Körpergewicht. Normalerweise sollten o.5 bis 4o mg, vorzugsweise 1.o bis 2o mg/kg/Tag in einer oder mehreren Einzeldosen ausreichen, um die gewünschte Besserung zu erreichen.

Die Verformbarkeit der Erythrozyten wird überlicherweise im sogenannten Filtrationstest gemessen. Dabei wird eine Suspension von Erythrozyten durch ein Filter geschickt, das Poren besitzt, die einen Durchmesser haben, der dem kleiner Kapillaren entspricht. Eine Substanz, die die Verformbarkeit der Erythrozyten verbessert, muß auch gleichzeitig die Passage der Erythrozyten durch diese Filterporen und damit die Filtrierbarkeit der Erythrozyten-Suspension erhöhen.

Im vorliegenden Fall wurde untersucht, inwieweit die Zugabe von Verbindungen der vorliegenden Anmeldung zur Suspension von Human-Erythrozyten, deren Verformbarkeit verändern. Die Human-Erythrozyten stammten von Patienten mit peripheren und cerebralen Durchblutungsstörungen und waren schlechter verformbar als Erythrozyten Gesunder.

Folgende Verbindungen wurden untersucht:

a) 1-Phenoxy-3-[2-(1.3.5-trimethyl-pyrazol-4-yl-amino)-ethylamino]-propan-2-ol

b) 1-(4-Hydroxyphenoxy)-3-[2-(morpholincarbonamido)-ethylamino]-propan-2-ol

c) 1-Phenoxy-3-[2-(1.3.5-trimethyl-pyrimidin-2.4-dion-6-yl-N-methylamino)ethylamino]-propan-2-ol

d) 1-Phenoxy-3-[2-(1-allyl-3.5-dimethyl-pyrazol-4-yl-amino)-ethylamino]-propan-2-ol

e) 1-Phenoxy-3-[2-(1-n-propyl-3.5-dimethyl-pyrazol-4-yl-amino)-ethylamino]-propan-2-ol

f) 1-(4-Hydroxyphenoxy)-3-[2-(1.3.5-trimethyl-pyrazol-4-yl-amino)-ethylamino]-propan-2-ol

g) 1-Phenoxy-3-[2-(1.4-dimethyl-pyrazol-5-yl-amino)-ethylamino]-propan-2-ol

Als Vergleichssubstanz wurde das handelsübliche Pentoxifyllin (Trental) = 3,7-Dihydro-3,7-dimethyl-1-(5-oxohexyl)-1H-purin-2,6-dion gewählt.

Methodenbeschreibung

Bestimmung der Erythrozytenfiltrierbarkeit

A. Methode nach DODDS und DORMANDY[1]

Blut mit pathologisch-rheologischen Eigenschaften (Verformbarkeitsindex kleiner 0,55) wurde wie folgt aufgearbeitet und filtriert:

Das mit Lithiumheparin anticoagulierte Blut wurde 15 min beim 3000 Umdrehungen/min zentrifugiert. Das Plasma wurde zunächst vorgefiltert durch Filter mit einer Porengröße von 1,2 µm, um Proteinpräzipitate zurückzuhalten. Nach Entfernen der buffy coat wurden die Erythrozyten im autologen Plasma resuspendiert, so daß ein Hämatokrit von 5 % entstand. Jeweils 1 ml davon wurde anschließend durch ein Nucleoporefilter mit einer Porengröße von 5 µm laufengelassen. Die Filtration wurde nach 60 sec abgebrochen. Anschließend wurde der Verformbarkeitsindex bestimmt. Das ist das Verhältnis zwischen der optischen Dichte des Filtrats und der der unfiltrierten Suspension. Entsprechend liegen alle Werte zwischen 0 und 1. Bei gesunden Spendern liegt der so erhaltene Index bei 0,63 (Standardabweichung 0,2).

B. Filtration mit der SER-Methode[2] (= selektierendes Erythrozyten-Rigidometer).

Patienten mit peripherer Durchblutungsstörung (Stadium II nach FONTAINE) wurde Blut entnommen und mit Natrium-Heparinat versetzt. Die Blute wurden zentrifugiert und die Plasmen in mehrere gleichgroße Portionen fraktioniert, wobei jeder Fraktion eine andere Substanz oder eine Substanz in verschiedenen Konzentrationen zugesetzt wurde.

Nach der Zugabe der Prüfsubstanzen wurden die Blutzellen resuspendiert und ein Hämatokritwert von 45 % eingestellt. Alle Blutproben wurden 60 min lang bei 37°C inkubiert. Um eine pH-Konstanz bei 7,4 zu gewährleisten, wurden die Proben während der Inkubationszeit mit Carbogen (80 % Sauerstoff und 20 % Kohlendioxyd) überschichtet. Anschließend wurde die Passagezeit von 250 Erythrozyten mit Hilfe des SER-Gerätes bestimmt. Dessen einzige Pore ist von definiertem Durchmesser und von definierter Länge. Der treibende Druck wird so gewählt, daß die resultierende Schubspannung im physiologischen Bereich bleibt. Als Vergleichsgröße wird die mittlere Erythrozyten-Passagezeit aller 250 Erythrozyten in msec bestimmt.

Tabelle

Zusammenstellung hämorheologischer Befunde

D + D = Methode nach DODDS und DORMANDY[1];
Meßwert = Verformbarkeitsindex (größerer Wert entspricht besserer Erythrozytenverformbarkeit)

SER = Selektierendes Erythrozyten-Rigidometer[2];
Meßwert = mittlere Passagezeit in msec (kleinerer Wert entspricht besserer Erythrozytenverformbarkeit)

| Substanz | Konz. (Mol/l) | Methode | Pat.zahl insgesamt | Mittelwert | davon rheolog. Verbesserg. | MW |
|---|---|---|---|---|---|---|
| a) | $10^{-7}$ | D + D | 10 | 0,48 | 4 | 0,48 |
| | $10^{-8}$ | D + D | 10 | 0,48 | 8 | 0,56 |
| | $10^{-9}$ | D + D | 10 | 0,48 | 10 | 0,57 |
| | $10^{-6}$ | SER | 10 | 20,24 | 7 | 19,48 |
| | $10^{-7}$ | SER | 10 | 20,24 | 9 | 18,68 |
| | $10^{-8}$ | SER | 10 | 20,24 | 9 | 19,02 |
| b) | $10^{-6}$ | D + D | 6 | 0,48 | 3 | 0,50 |
| | $10^{-8}$ | D + D | 6 | 0,48 | 5 | 0,52 |
| c) | $10^{-8}$ | SER | 3 | 17,97 | 2 | 17,20 |
| d) | $10^{-8}$ | SER | 3 | 17,97 | 2 | 16,57 |
| e) | $10^{-8}$ | SER | 3 | 17,97 | 3 | 16,93 |
| f) | $10^{-8}$ | SER | 3 | 17,97 | 1 | 17,90 |
| g) | $10^{-8}$ | SER | 3 | 17,97 | 1 | 18,13 |
| Pentoxifyllin (Vergleichssubstanz) | $0,9x10^{-5}$ | D + D | 10 | 0,48 | 5 | 0,49 |
| | $0,9x10^{-5}$ | D + D | 6 | 0,48 | 4 | 0,51 |
| | $1,8x10^{-5}$ | SER | 10 | 20,24 | 10 | 18,13 |

## Ergebnisse

Die Ergebnisse in der Tabelle zeigen, daß die aufgelisteten Substanzen zu einer Verbesserung der Erythrozyten-Verformbarkeit führen. Für die Verbindung a) und Pentoxifyllin konnte dieser Effekt sogar mit zwei unterschiedlichen Methoden festgestellt werden.

Diese Effekte zeigen, daß die untersuchten Substanzen sich demzufolge für die Behandlung von peripheren und cerebralen Durchblutungsstörungen und anderen Erkrankungen, die mit Veränderung der Fließeigenschaften einhergehen, eignen.

## Literaturverzeichnis

1) DODDS, A. J.; M. J. G. O'REILLY; C. J. P. YATES; L. T. COTTON; P. T. FLUITE; J. A. DORMANDY: Haemorrheological response to plasma exchange in Raynaud's syndrome Br. Med. J. (1979), 2, 1186-1187.

2) ROGGENKAMP, H. G.; F. JUNG; H. KIESEWETTER: Ein Gerät zur elektrischen Messung der Verformbarkeit von Erythrozyten. Biomed. Tech. (1983), 28, 100-104.

## Patentansprüche

1. Verwendung von Alkylendiaminen der allgemeinen Formel I

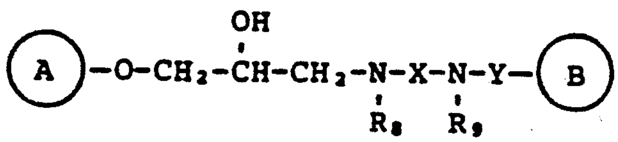 (I),

in der

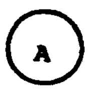 entweder

1. einen Phenylrest der allgemeinen Formel II

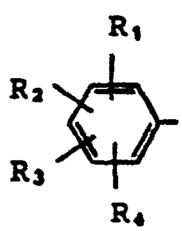 (II),

in welcher $R_1$, $R_2$, $R_3$ und $R_4$ gleich oder verschieden sind und Wasserstoff, Niederalkyl, Cyan, Carboxamido, Halogen, Hydroxy, Niederacyloxy, Niederalkoxy, Methoxyniederalkoxy, Niederalkenyloxy, Phenylniederalkoxy, Amino oder Niederacylamino bedeuten

oder

2. einen bi- oder tricyclischen, gegebenenfalls teilweise hydrierten heteroaromatischen Rest der allgemeinen Formel III

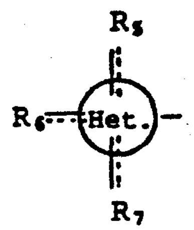 (III),

in welcher $R_5$, $R_6$, $R_7$ gleich oder verschieden sind und Wasserstoff, Niederalkyl, Benzyl, Niederalkanoyl, Cyan, Hydroxymethyl, Niederalkoxycarbonyl, Carbamoyl und die zweibindigen Reste Sauerstoff oder Schwefel bedeuten,

$R_8$, $R_9$ gleich oder verschieden sind und Wasserstoff oder Niederalkyl bedeuten,

X eine geradkettige oder verzweigte Alkylenkette mit bis zu 6 Kohlenstoffatomen ist,

Y ein Valenzstrich oder die >C=O-Gruppe ist und

(B) entweder

1. einen Phenylrest der allgemeinen Formel IV

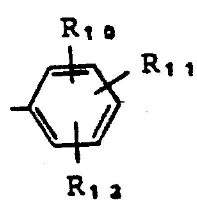 (IV),

in welcher $R_{10}$, $R_{11}$ und $R_{12}$ gleich oder verschieden sind und Wasserstoff, Halogen, Niederalkyl, Niederalkoxy, Niederalkylmercapto, Nitro, Amino, Niederacylamine oder $R_{10}$ und $R_{11}$ gemeinsam eine gegebenenfalls ungesättigte Trimethylenkette oder eine Alkylendioxy-Gruppe bedeuten

oder

2. einen mono-, bi- oder tricyclischen heteroaromatischen oder hydroheteroaromatischen Rest der allgemeinen Formel V

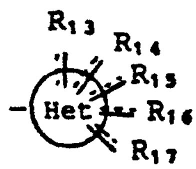

(V),

in welcher $R_{13}$, $R_{14}$, $R_{15}$, $R_{16}$, $R_{17}$ gleich oder verschieden sind und ein- oder zweibindige Substituenten bedeuten, und zwar Wasserstoff, Halogen, Niederalkyl, Allyl, Niederalkoxy, Allyloxy, Nitro, Amino, Niederacylamino, Cyano, Phenylniederalkyl, gegebenenfalls substituiertes Phenyl, Sauerstoff oder Schwefel,

oder

3. einen Aminrest der allgemeinen Formel VI

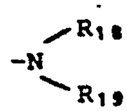

(VI),

in welcher $R_{18}$ und $R_{19}$ gleich oder verschieden sind und Wasserstoff, Niederalkyl, Hydroxyniederalkyl, Alkoxyniederalkyl, Phenyl oder gemeinsam mit dem Stickstoffatom einen gegebenenfalls durch Heteroatome wie Sauerstoff, Schwefel oder die Gruppe $>N-R_{20}$ unterbrochenen Ring,

$R_{20}$ Niederalkyl, gegebenenfalls substituiertes Phenyl, Niederalkanoyl oder gegebenenfalls substituiertes Benzoyl

darstellen

sowie deren pharmakologisch verträglichen Salze zur Herstellung von Arzneimitteln zur Behandlung von Durchblutungsstörungen.

2) Verwendung von Verbindungen gemäß Anspruch 1, in denen

A einen Phenyl-Rest, der gegebenenfalls durch Hydroxy substituiert ist oder einen Indazol-, Indol-, Benzimidazol- oder Benztriazol-Rest,

$R_8$, $R_9$ Wasserstoff oder Methyl,

X Ethylen,

Y Valenz oder eine >CO-Gruppe und

B einen Phenyl-Rest, der gegebenenfalls durch Methylgruppen substituiert ist, einen Pyrazol-Rest, der ein- bis dreifach durch Methyl, Propyl oder Allyl substituiert sein kann, oder
einen Pyrimidin-2.4-dion-Rest, der ein- bis dreifach durch Methyl substituiert sein kann,

bedeuten.

3) Verwendung von 1 Phenoxy-3-[2-(1.3.5-trimethyl-pyrazol-4-yl-amino)-ethylamino]-propan-2-ol gemäß Anspruch 1.

4) Verwendung von 1-(4-Hydroxyphenoxy)-3-[2-(morpholin-carbonamido)-ethylamino]-propan-2-ol gemäß Anspruch 1.

5) Verwendung von 1-Phenoxy-3-[2-(1.3.5-trimethyl-pyrimidin-2.4-dion-6-yl-N-methylamino)-ethylamino]-propan-2-ol gemäß Anspruch 1.

6) Verwendung von 1-Phenoxy-3-[2-(1-allyl-3.5-dimethyl-pyrazol-4-yl-amino)-ethylamino]-propan-2-ol gemäß Anspruch 1.

7) Verwendung von 1-Phenoxy-3-/2-(1-n-propyl-3.5-dimethyl-pyrazol-4-yl-amino)-ethylamino7-propan-2-ol gemäß Anspruch 1.

8) Verwendung von 1-(4-Hydroxyphenoxy)-3-/2-(1.3.5-trimethyl-pyrazol-4-yl-amino)-ethylamino7-propan-2-ol gemäß Anspruch 1.

9) Verwendung von 1-Phenoxy-3-/2-(1.4-dimethyl-pyrazol-5-yl-amino)-ethylamino7-propan-2-ol gemäß Anspruch 1.

10) Verwendung von 1-(Indazol-4-yloxy)-3-/2-(2.6-dimethyl-phenylamino)-ethylamino7-propan-2-ol gemäß Anspruch 1.

11) Arzneimittel zur Behandlung von Durchblutungsstörungen, enthaltend eine Verbindung gemäß einem der Ansprüche 1 bis 10 und übliche Träger- und Hilfsstoffe.